(19) Europäisches Patentamt
European Patent Office
Office européen des brevets

(11) EP 2 036 893 A1

(12) **EUROPEAN PATENT APPLICATION**
published in accordance with Art. 153(4) EPC

(43) Date of publication:
**18.03.2009 Bulletin 2009/12**

(21) Application number: **07767882.9**

(22) Date of filing: **29.06.2007**

(51) Int Cl.:
***C07D 231/56*** (2006.01) ***A61K 31/416*** (2006.01)
***A61K 31/4439*** (2006.01) ***A61K 31/454*** (2006.01)
***A61K 31/496*** (2006.01) ***A61K 31/5377*** (2006.01)
***A61P 35/00*** (2006.01) ***A61P 35/02*** (2006.01)
***A61P 43/00*** (2006.01) ***C07D 401/06*** (2006.01)
***C07D 403/10*** (2006.01)

(86) International application number:
**PCT/JP2007/063095**

(87) International publication number:
**WO 2008/001885 (03.01.2008 Gazette 2008/01)**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(30) Priority: **30.06.2006 JP 2006180530**

(71) Applicant: **Kyowa Hakko Kirin Co., Ltd.**
**Tokyo 100-8185 (JP)**

(72) Inventors:
- **SHIOTSU, Yukimasa,**
**Nagaizumi-cho,Sunto-gun,Shizuoka 411-8731 (JP)**
- **ISHII, Kenichi**
**Nagaizumi-cho,Sunto-gun,Shizuoka 411-8731 (JP)**
- **UMEHARA, Hiroshi**
**Nagaizumi-cho,Sunto-gun,Shizuoka 411-8731 (JP)**
- **AKINAGA, Shiro**
**Nagaizumi-cho,Sunto-gun,Shizuoka 411-8731 (JP)**

(74) Representative: **Harding, Charles Thomas et al**
**D Young & Co**
**120 Holborn**
**London EC1N 2DY (GB)**

(54) **Abl KINASE INHIBITOR**

(57) The present invention provides an Abelson kinase (Abl kinase) inhibitor which comprises, as an active ingredient, an indazole derivative represented by Formula (I) (wherein $R^1$ represents substituted or unsubstituted aryl or a substituted or unsubstituted heterocyclic group) or a pharmaceutically acceptable salt thereof,
an Abl kinase inhibitor which comprises, as an active ingredient, an indazole derivative represented by Formula (Ia) [wherein $R^2$ represents $CONR^{4a}R^{4b}$ (wherein $R^{4a}$ and $R^{4b}$ may be the same or different and each represents a hydrogen atom, substituted or unsubstituted lower alkyl, or the like, or $R^{4a}$ and $R^{4b}$ are combined together with the adjacent nitrogen atom thereto to form a substituted or unsubstituted heterocyclic group) or the like, and $R^3$ represents a hydrogen atom, substituted or unsubstituted lower alkyl, or the like] or a pharmaceutically acceptable salt thereof, and the like.

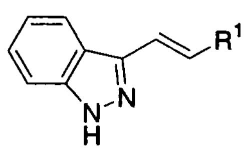
(I)

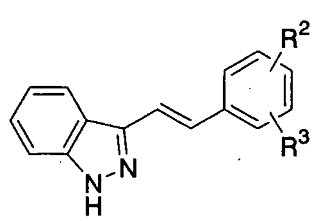
(Ia)

EP 2 036 893 A1

## Description

### Technical Field

The present invention relates to an Abelson kinase (Abl kinase) inhibitor which comprises, as an active ingredient, an indazole derivative or a pharmaceutically acceptable salt thereof, and the like.

### Background Art

Bcr-Abl is a fusion protein produced by fusion of Abl kinase and BCR gene through translocation of Philadelphia chromosome (Ph chromosome) and is a molecule identified as a causative gene product in chronic myeloid leukemia (CML) and acute lymphoblastic leukemia (ALL) [Nature, Vol. 315, p. 550 (1985), Vol. 325, p. 631 (1987)]. Bcr-Abl is a tyrosine kinase which controls oncogenic transformation and infinite proliferation of Ph-positive CML and ALL cells, and imatinib which selectively inhibits Abl kinase is clinically applied as a medicine having low toxicity to CML and ALL patients and exhibiting a high clinical effect [New England Journal of Medicine, Vol. 345, p. 645 (2002)]. Further, an Abl kinase inhibitor is thought to be effective as a therapeutic agent for various cancers other than CML and ALL.

In addition, it has recently been reported that drug resistance to imatinib occurs in CML and ALL treatment with imatinib, and a decrease in sensitivity to imatinib due to the occurrence of point mutations in kinase domains of Bcr-Abl has been reported as one of the mechanism of the drug resistance [Nature Review Drug Discovery, Vol. 3, p. 1001 (2004)]. Therefore, an inhibitor to Bcr-Abl having point mutations and low sensitivity to imatinib is useful for leukemia having acquired resistance to imatinib. As one of the point mutations of Bcr-Abl which are detected at the highest frequency in imatinib-resistant CML and ALL patients, there has been reported the gene mutation (T315I mutation) in which a threonine residue at the 315-position is substituted by isoleucine [Science, Vol. 293, p. 876 (2001)], and an inhibitor to leukemia cells having the Abl T315I mutation is useful for leukemia having acquired resistance to imatinib.

As the Abl-kinase inhibitor other than imatinib, AMN107 [Cancer Cell, Vol. 7, p. 129 (2005)], BMS-354825 [Science, Vol. 305, p. 399 (2004)], and the like have been known. It has been reported that AMN107 and BMS-354825 exhibit an inhibitory action on growth of leukemia cells having several point mutations detected in patients with imatinib-resistant leukemia, but both medicines have low sensitivity to leukemia cells having Abl T315I mutation [Cancer Research, Vol. 65, p. 4500 (2005)]. On the other hand, VX-680 and BIRB-796 have been reported as a medicine exhibiting an inhibitory action on leukemia cells having the Abl T315I mutation [Proceeding of the national academy of science of the united states of America, Vol. 102, p. 101011 (2005)].

Further, various compounds have been reported as indazole derivatives (for example, refer to Patent Documents 1 to 12 and Non-patent Document 1).

Patent Document 1: Japanese Published Unexamined Patent Application (Kokai) No. 32059/1990
Patent Document 2: WO01/53268
Patent Document 3: WO02/10137
Patent Document 4: WO01/02369
Patent Document 5: WO02/083648
Patent Document 6: WO03/101968
Patent Document 7: WO2004/094388
Patent Document 8: WO2004/050088
Patent Document 9: WO2005/0137171
Patent Document 10: WO2005/012257
Patent Document 11: W02005/012258
Patent Document 12: WO2005/094823
Non-Patent Document 1: Khimiya Geterotsiklicheskikh Soedinenii, vol. 7, pages 957-959 (1978)

### Disclosure of the Invention

### Problems to be solved by the Invention

An object of the present invention is to provide an Abl kinase inhibitor which comprises, as an active ingredient, an indazole derivative or a pharmaceutically acceptable salt thereof and the like.

### Means for Solving the Problems

The present invention relates to following (1) to (46).

(1) An Abl kinase inhibitor which comprises, as an active ingredient, an indazole derivative represented by Formula (I)

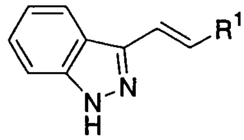

(I)

(wherein $R^1$ represents substituted or unsubstituted aryl or a substituted or unsubstituted heterocyclic group) or a pharmaceutically acceptable salt thereof.

(2) An Abl kinase inhibitor which comprises, as an active ingredient, an indazole derivative represented by Formula (Ia)

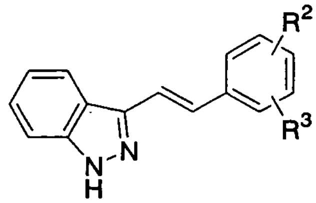

(Ia)

[wherein $R^2$ represents $CONR^{4a}R^{4b}$ (wherein $R^{4a}$ and $R^{4b}$ may be the same or different and each represents a hydrogen atom, substituted or unsubstituted lower alkyl, substituted or unsubstituted aryl, substituted or unsubstituted aralkyl, or a substituted or unsubstituted heterocyclic group, or $R^{4a}$ and $R^{4b}$ are combined together with the adjacent nitrogen atom thereto to form a substituted or unsubstituted heterocyclic group) or $NR^{5a}R^{5b}$ (wherein $R^{5a}$ represents substituted or unsubstituted lower alkylsulfonyl or substituted or unsubstituted arylsulfonyl and $R^{5b}$ represents a hydrogen atom or substituted or unsubstituted lower alkyl) and $R^3$ represents a hydrogen atom, halogen, cyano, nitro, hydroxy, carboxy, lower alkoxycarbonyl, substituted or unsubstituted lower alkyl, substituted or unsubstituted lower alkoxy, substituted or unsubstituted lower alkanoyl, $CONR^{6a}R^{6b}$ (wherein $R^{6a}$ and $R^{6b}$ may be the same or different and each represents a hydrogen atom, substituted or unsubstituted lower alkyl, substituted or unsubstituted aryl, substituted or unsubstituted aralkyl or a substituted or unsubstituted heterocyclic group, or $R^{6a}$ and $R^{6b}$ are combined together with the adjacent nitrogen atom thereto to form a substituted or unsubstituted heterocyclic group) or $NR^{7a}R^{7b}$ (wherein $R^{7a}$ and $R^{7b}$ may be the same or different and each represents a hydrogen atom, substituted or unsubstituted lower alkyl, substituted or unsubstituted lower alkanoyl, substituted or unsubstituted aroyl, substituted or unsubstituted heteroaroyl, substituted or unsubstituted aralkyl, substituted or unsubstituted lower alkylsulfonyl or substituted or unsubstituted arylsulfonyl)], or a pharmaceutically acceptable salt thereof.

(3) The Abl kinase inhibitor according to (2), wherein $R^2$ is $CONR^{4a}R^{4b}$ (wherein $R^{4a}$ and $R^{4b}$ have the same meanings as defined above, respectively) and $R^3$ is a hydrogen atom.

(4) The Abl kinase inhibitor according to (2), wherein $R^2$ is $NR^{5a}R^{5b}$ (wherein $R^{5a}$ and $R^{5b}$ have the same meanings as defined above, respectively) and $R^3$ is substituted or unsubstituted lower alkoxy.

(5) The Abl kinase inhibitor according to (2), wherein $R^2$ is $NR^{5a}R^{5b}$ (wherein $R^{5a}$ and $R^{5b}$ have the same meanings as defined above, respectively) and $R^3$ is a hydrogen atom.

(6) An Abl kinase inhibitor which comprises, as an active ingredient, an indazole derivative represented by Formula (Ib)

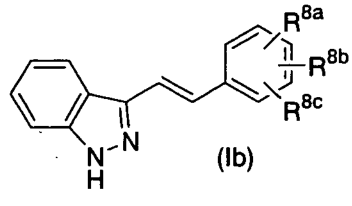

(Ib)

[wherein $R^{8a}$, $R^{8b}$ and $R^{8c}$ may be the same or different and each represents a hydrogen atom, halogen, nitro, nitroso, carboxy, cyano, substituted or unsubstituted lower alkyl, substituted or unsubstituted lower alkanoyl, substituted or unsubstituted lower alkoxycarbonyl, substituted or unsubstituted aryl, $NR^{9a}R^{9b}$ (wherein $R^{9a}$ and $R^{9b}$ may be the same or different and each represents a hydrogen atom, substituted or unsubstituted lower alkyl, substituted or unsubstituted lower alkenyl, substituted or unsubstituted lower alkynyl, substituted or unsubstituted lower alkoxy, substituted or unsubstituted lower alkanoyl, substituted or unsubstituted aryl, substituted or unsubstituted aroyl, a substituted or unsubstituted heterocyclic group or substituted or unsubstituted heteroaroyl, or $R^{9a}$ and $R^{9b}$ are combined together with the adjacent nitrogen atom thereto to form a substituted or unsubstituted heterocyclic group) or $OR^{10}$ (wherein $R^{10}$ represents a hydrogen atom, substituted or unsubstituted lower alkyl, substituted or unsubstituted aryl, substituted or unsubstituted aroyl or a substituted or unsubstituted heterocyclic group)], or a pharmaceutically acceptable salt thereof.

(7) The Abl kinase inhibitor according to (6), wherein at least one of $R^{8a}$, $R^{8b}$ and $R^{8c}$ is $NR^{9a}R^{9b}$ (wherein $R^{9a}$ and $R^{9b}$ have the same meanings as defined above, respectively).

(8) The Abl kinase inhibitor according to (6), wherein at least one of $R^{8a}$, $R^{8b}$ and $R^{8c}$ is $NR^{9c}R^{9d}$ (wherein $R^{9c}$ and $R^{9d}$ may be the same or different and each represents a hydrogen atom or substituted or unsubstituted lower alkanoyl, or $R^{9c}$ and $R^{9d}$ are combined together with the adjacent nitrogen atom thereto to form a substituted or unsubstituted heterocyclic group).

(9) The Abl kinase inhibitor according to (6), wherein at least one of $R^{8a}$, $R^{8b}$ and $R^{8c}$ is $OR^{10}$ (wherein $R^{10}$ has the same meaning as defined above).

(10) The Abl kinase inhibitor according to (6), wherein at least one of $R^{8a}$, $R^{8b}$ and $R^{8c}$ is $OR^{10a}$ (wherein $R^{10a}$ represents substituted or unsubstituted lower alkyl).

(11) An Abl kinase inhibitor which comprises, as an active ingredient, an indazole derivative represented by Formula (Ib-1)

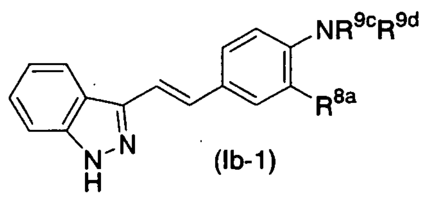

(Ib-1)

(wherein $R^{8a}$, $R^{9c}$ and $R^{9d}$ have the same meanings as defind above, respectively) or a pharmaceutically acceptable salt thereof.

(12) An Abl kinase inhibitor which comprises, as an active ingredient, an indazole derivative represented by Formula (Ib-2)

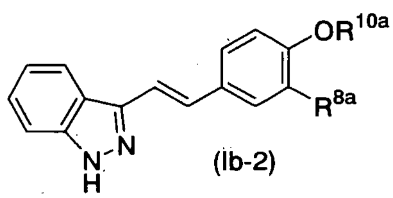

(Ib-2)

(wherein $R^{8a}$ and $R^{10a}$ have the same meanings as defind above, respectively) or a pharmaceutically acceptable salt thereof.

(13) An Abl kinase inhibitor which comprises, as an active ingredient, an indazole derivative represented by Formula (Ic)

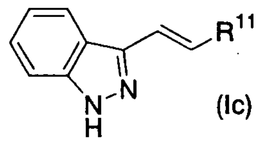

(Ic)

{wherein $R^{11}$ represents a substituted or unsubstituted heterocyclic group [substituent(s) in the substituted heterocyclic group may be the same or different, are 1 to 3 in number, and are oxo, formyl, carboxy, lower alkoxycarbonyl, substituted or unsubstituted lower alkyl, substituted or unsubstituted lower alkoxy, $CONR^{12a}R^{12b}$ (wherein $R^{12a}$ and

$R^{12b}$ may be the same or different and each represents a hydrogen atom or substituted or unsubstituted lower alkyl), $NR^{13a}R^{13b}$ (wherein $R^{13a}$ and $R^{13b}$ may be the same or different and each represents a hydrogen atom, lower alkanoyl, lower alkoxycarbonyl, aralkyl, substituted or unsubstituted lower alkyl, substituted or unsubstituted aryl, substituted or unsubstituted aroyl or a substituted or unsubstituted heterocyclic group) or - $O(CR^{14a}R^{14b})_nO$- (wherein $R^{14a}$ and $R^{14b}$ may be the same or different and each represents a hydrogen atom or lower alkyl, n represents 2 or 3, and two terminal oxygen atoms are bonded to the same carbon atom in the heterocyclic group of the substituted heterocyclic group)]} or a pharmaceutically acceptable salt thereof.

(14) The Abl kinase inhibitor according to (13), wherein substituents in the substituted heterocyclic group are amino, oxo, lower alkoxy, lower alkoxycarbonylamino, aroylamino or lower alkoxycarbonyl-substituted lower alkyl.

(15) The Abl kinase inhibitor according to (13), wherein $R^{11}$ is 3-pyridyl.

(16) A therapeutic agent for disease associated with Abl kinase which comprises, as an active ingredient, the indazole derivative or the pharmaceutically acceptable salt thereof described in any one of (1) to (15).

(17) The therapeutic agent according to (16), wherein Abl kinase is Abl kinase which has point mutation in the amino acid sequence thereof.

(18) The therapeutic agent according to (16), wherein Abl kinase is Abl kinase which has gene mutation in which a threonine residue at the 315-position is substituted with isoleucine in the amino acid sequence thereof (Abl T315I).

(19) The therapeutic agent according to any one of (16) to (18), wherein disease associated with Abl kinase is cancer.

(20) The therapeutic agent according to (19), wherein cancer is cancer derived from hematopoietic tumor, mammary cancer, uterine body cancer, uterine cervix cancer, prostatic cancer, bladder cancer, renal cancer, gastric cancer, esophageal cancer, hepatic cancer, biliary tract cancer, colon cancer, rectal cancer, pancreatic cancer, lung cancer, oral cavity and pharynx cancer, osteosarcoma, melanoma or brain neoplasm.

(21) The therapeutic agent for cancer according to (19), wherein cancer is leukemia, myeloma or lymphoma.

(22) The therapeutic agent for cancer according to (19), wherein cancer is chronic myeloid leukemia.

(23) The therapeutic agent for cancer according to (19), wherein cancer is leukemia having acquired resistance to imatinib.

(24) The therapeutic agent for cancer according to (19), wherein cancer is chronic myeloid leukemia having acquired resistance to imatinib.

(25) A method for inhibiting Abelson kinase (Abl kinase) comprising a step of administering an effective amount of the indazole derivative or the pharmaceutically acceptable salt thereof described in any one of (1) to (15).

(26) A method for treating a disease associated with Abelson kinase (Abl kinase) comprising a step of administering an effective amount of the indazole derivative or the pharmaceutically acceptable salt thereof described in to any one of (1) to (15).

(27) The treating method according to (26), wherein Abelson kinase (Abl kinase) is Abl kinase which has point mutation in the amino acid sequence thereof.

(28) The treating method according to (26), wherein Abelson kinase (Abl kinase) is Abl kinase which has gene mutation in which a threonine residue at the 315-position is substituted with isoleucine in the amino acid sequence thereof (Abl T315I).

(29) The method for treating cancer comprising a step of administering an effective amount of the indazole derivative or the pharmaceutically acceptable salt thereof described in any one of (1) to (15).

(30) The treating method according to (29), wherein cancer is cancer derived from hematopoietic tumor, mammary cancer, uterine body cancer, uterine cervix cancer, prostatic cancer, bladder cancer, renal cancer, gastric cancer, esophageal cancer, hepatic cancer, biliary tract cancer, colon cancer, rectal cancer, pancreatic cancer, lung cancer, oral cavity and pharynx cancer, osteosarcoma, melanoma or brain neoplasm.

(31) The treating method according to (29), wherein cancer is leukemia, myeloma or lymphoma.

(32) The treating method according to (29), wherein cancer is chronic myeloid leukemia.

(33) The treating method according to (29), wherein cancer is leukemia having acquired resistance to imatinib.

(34) The treating method according to (29), wherein cancer is chronic myeloid leukemia having acquired resistance to imatinib.

(35) Use of the indazole derivative or the pharmaceutically acceptable salt thereof described in any one of (1) to (15) for the manufacture of Abelson kinase (Abl kinase) inhibitor.

(36) Use of the indazole derivative or the pharmaceutically acceptable salt thereof described in any one of (1) to (15) for the manufacture of therapeutic agent for disease associated with Abelson kinase (Abl kinase) inhibitor.

(37) The use according to (36), wherein Abelson kinase (Abl kinase) is Abl kinase which has point mutation in the amino acid sequence thereof.

(38) The use according to (36), wherein Abelson kinase (Abl kinase) is Abl kinase which has gene mutation in which a threonine residue at the 315-position is substituted with isoleucine in the amino acid sequence thereof (Abl T315I).

(39) Use of the indazole derivative or the pharmaceutically acceptable salt thereof described in any one of (1) to (15) for the manufacture of a therapeutic agent for cancer.

(40) The use according to (39), wherein cancer is cancer derived from hematopoietic tumor, mammary cancer, uterine body cancer, uterine cervix cancer, prostatic cancer, bladder cancer, renal cancer, gastric cancer, esophageal cancer, hepatic cancer, biliary tract cancer, colon cancer, rectal cancer, pancreatic cancer, lung cancer, oral cavity and pharynx cancer, osteosarcoma, melanoma or brain neoplasm.
(41) The use according to (39), wherein cancer is leukemia, myeloma or lymphoma.
(42) The use according to (39), wherein cancer is chronic myeloid leukemia.
(43) The use according to (39), wherein cancer is leukemia having acquired resistance to imatinib.
(44) The use according to (39), wherein cancer is chronic myeloid leukemia having acquired resistance to imatinib.
(45) An inhibitor against Abl kinase which has point mutation in the amino acid sequence thereof which comprises, as an active ingredient, the indazole derivative or the pharmaceutically acceptable salt thereof described in any one of (1) to (15).
(46) An inhibitor against Abl kinase which has gene mutation in which a threonine residue at the 315-position is substituted with isoleucine in the amino acid sequence thereof (Abl T315I kinase) which comprises, as an active ingredient, the indazole derivative or the pharmaceutically acceptable salt thereof described in any one of (1) to (15).

## Effect of the Invention

The present invention provides an abl kinase inhibitor which comprises, as an active ingredient, an indazole derivative or a pharmaceutically acceptable salt thereof, and the like.

## Best Mode for Carrying Out the Invention

Hereinafter, compounds represented by Formula (I), (Ia), (Ib), (Ib-1), (Ib-2) and (Ic) are referred to as Compound (I), (Ia), (Ib), (Ib-1), (Ib-2) and (Ic), respectively. The same shall apply to the compounds of the other formula numbers.
In the definitions for each groups in Formula (I), (Ia), (Ib), (Ib-1), (Ib-2) and (Ic):

(i) The halogen includes fluorine, chlorine, bromine, and iodine atoms.
(ii) Examples of the lower alkyl and the lower alkyl moieties of the lower alkoxy, the lower alkoxycarbonyl, the lower alkoxycarbonylamino, the lower alkoxycarbonyl-substituted lower alkyl and the lower alkylsulfonyl include, for example, linear, branched or cyclic alkyl or alkyl consisting of these alkyls in combination, having 1 to 10 carbon atom (s). More specific examples thereof are as follows.

(ii-a) Examples of the linear or branched lower alkyl include, for example, methyl, ethyl, *n*-propyl, isopropyl, *n*-butyl, isobutyl, *sec*-butyl, *tert*-butyl, *n*-pentyl, neopentyl, *n*-hexyl, *n*-heptyl, *n*-octyl, *n*-nonyl, *n*-decyl and the like;
(ii-b) examples of the cyclic lower alkyl include, for example, cyclopropyl, cyclobutyl, cyclopentyl, cyclohexyl, cycloheptyl, cyclooctyl, cyclodecyl, noradamantyl, adamantyl, bicyclo[2.2.1]heptyl, bicyclo[2.2.2]octyl, bicyclo [3.3.0]octyl, bicyclo[3.3.1]nonyl and the like; and
(ii-c) examples of the lower alkyl consisting of linear or branched alkyl and cyclic alkyl in combination include, for example, cyclopropylmethyl, cyclopentylmethyl, cyclooctylethyl and the like.

(iii) The alkylene moiety of the lower alkoxycarbonyl-substituted lower alkyl and the aralkyl has the same meaning as the group formed by removing one hydrogen atom from the linear or branched lower alkyl (ii-a) defined above.
(iv) Examples of the lower alkenyl include, for example, linear or branched alkenyl having 2 to 10 carbon atoms such as vinyl, allyl, 1-propenyl, 1-butenyl, 3-butenyl, 2-pentenyl, 4-pentenyl, 2-hexenyl, 5-hexenyl, 2-decenyl or 9-decenyl.
(v) Examples of the lower alkynyl include, for example, linear or branched alkynyl having 2 to 10 carbon atoms such as ethynyl, 2-propynyl, 3-butynyl, 4-pentynyl, 5-hexynyl or 9-decynyl.
(vi) Examples of the aryl and the aryl moieties of the aralkyl, the aroyl, the aroylamino and the arylsulfonyl include, for example, monocyclic aryl or fused aryl in which two or more rings are fused, and more specific examples include aryl having 6 to 14 carbon atoms as ring-constituting members, such as phenyl, naphthyl, indenyl or anthranyl.
(vii) Examples of the lower alkanoyl include, for example, linear, branched, or cyclic lower alkanyol, or lower alkanoyl consisting of these lower alkanoyls in combination, having 1 to 8 carbon atom(s), such as formyl, acetyl, propionyl, butyryl, isobutyryl, valeryl, isovaleryl, pivaloyl, hexanoyl, heptanoyl, octanoyl, cyclopropylcarbonyl, cyclobutylcarbonyl, cyclopropylcarbonyl, cyclopropylmethylcarbonyl, cyclohexylcarbonyl, 1-methylcyclopropylcarbonyl or cycloheptylcarbonyl.
(viii) Examples of the heterocyclic group include, for example, heteroaromatic group, heteroalicyclic group and the like.
(viii-a) Examples of the heteroaromatic group include, for example, monocyclic heteroaromatic group, fused heteroaromatic group in which two or more rings are fused or the like. The type and number of the heteroatom contained in heteroaromatic group are not specifically limited and the heteroaromatic group may contain, for example, one or more heteroatoms selected from the group consisting of a nitrogen atom, a sulfur atom and an oxygen atom. More specific examples include heteroaromatic group having 5 to 14 atoms as ring-constituting members, such as furyl, thienyl, pyrrolyl, imidazolyl, pyrazolyl, triazolyl, tetrazolyl, oxazolyl, oxadiazolyl, thiazolyl, pyridyl, pyrazinyl, pyrimidinyl, pyridazinyl, triazinyl, indolyl, indazolyl, benzimidazolyl, benzoxazolyl, benzothiazolyl, quinolyl, isoquinolyl, phthalazinyl, naphthyridinyl, quinoxalinyl, quinazolinyl, cinnolinyl, purinyl or coumarinyl.

(viii-b) Examples of the heteroalicyclic group include, for example, monocyclic heteroalicyclic group or fused heteroalicyclic group in which two or more rings are fused. The type and number of the heteroatom contained in heteroalicyclic groups are not specifically limited and the heteroalicyclic group may contain, for example, one or more heteroatoms selected from the group consisting of a nitrogen atom, a sulfur atom and an oxygen atom. More specific examples include, for example, pyrrolidinyl, 2,5-dioxopyrrolidinyl, thiazolidinyl, oxazolidinyl, piperidyl, 1,2-dihydropyridyl, piperazinyl, homopiperazinyl, morpholinyl, thiomorpholinyl, pyrazolinyl, oxazolinyl, dioxolanyl, tetrahydropyranyl, tetrahydrothiopyranyl, tetrahydrofuryl, tetrahydroquinolyl, tetrahydroisoquinolyl, tetrahydroquinoxalinyl, octahydroquinolyl, dihydroindolyl, 1,3-dioxoisoindolinyl and the like.

(ix) Examples of the heterocyclic group formed together with the adjacent nitrogen atom include 5- or 6-membered monocyclic heterocyclic group containing at least one nitrogen atom (the monocyclic heterocyclic group may further contain any other of a nitrogen atom, an oxygen atom and a sulfur atom) and bicyclic or tricyclic fused heterocyclic group containing at least one nitrogen atom in which 3- to 8-membered rings are fused (the fused heterocyclic group may further contain any other of a nitrogen atom, an oxygen atom and a sulfur atom). More specific examples include, for example, pyrrolidinyl, piperidino, piperazinyl, morpholino, thiomorpholino, homopiperidino, homopiperazinyl, tetrahydropyridyl, tetrahydroquinolyl, tetrahydroisoquinolyl and the like.

(x) The heteroaryl moiety in the heteroaroyl has the same meaning as the heteroaromatic group (viii-a) defined above.

(xi) Examples of the substituents in the substituted lower alkyl, the substituted lower alkoxy, the substituted lower alkenyl, the substituted lower alkynyl, the substituted lower alkanoyl, the substituted lower alkoxycarbonyl and the substituted lower alkylsulfonyl which may be the same or different and in number of 1 to 3, include

(xi-a) hydroxy,
(xi-b) lower alkoxy,
(xi-c) oxo,
(xi-d) carboxy,
(xi-e) lower alkoxycarbonyl,
(xi-f) heteroaroyl,
(xi-g) arylsulfonyl,
(xi-h) substituted or unsubstituted aryl (the substituent(s) in the substituted aryl is carboxy, lower alkoxy, lower alkoxycarbonyl or the like),
(xi-i) a substituted or unsubstituted heterocyclic group (the substituent(s) in the substituted heterocyclic group is carboxy, lower alkoxy, lower alkoxycarbonyl or the like),
(xi-j) $CONR^{15a}R^{15b}$ {wherein $R^{15a}$ and $R^{15b}$ may be the same or different and each represents a hydrogen atom or substituted or unsubstituted lower alkyl [the substituent(s) in the substituted lower alkyl, which is 1 to 3 in number, is for example, halogen, hydroxy, oxo, nitro, cyano, carboxy, lower alkanoyl, lower alkoxycarbonyl, aroyl or substituted or unsubstituted lower alkoxy (the substituent(s) in the substituted lower alkoxy, which is 1 to 3 in number, is for example, hydroxy or the like) or the like] or $R^{15a}$ and $R^{15b}$ are combined together with the adjacent nitrogen atom thereto to form a substituted or unsubstituted heterocyclic group [the substituent(s) in the substituted heterocyclic group formed together with the adjacent nitrogen atom, which is 1 to 3 in number, is for example, halogen, hydroxy, oxo, nitro, cyano, carboxy, lower alkanoyl, lower alkoxycarbonyl, aralkyl, aroyl, substituted or unsubstituted lower alkyl (the substituent(s) in the substituted lower alkyl, which is 1 to 3 in number, is for example, hydroxy or the like), substituted or unsubstituted lower alkoxy (the substituent(s) in the substituted lower alkoxy, which is 1 to 3 in number, is for example, hydroxy or the like) or the like]},
(xi-k) $NR^{16a}R^{16b}$ (wherein $R^{16a}$ and $R^{16b}$ have the same meanings as $R^{15a}$ and $R^{15b}$ defined above, respectively),
(xi-l) lower alkanoylamino,
(xi-m) N-(lower alkanoyl)-N-(lower alkyl)amino, and the like.

In the definition of the substituents (xi) in the substituted lower alkyl, the substituted lower alkoxy, the substituted lower alkenyl, the substituted lower alkynyl, the substituted lower alkanoyl, the substituted lower alkoxycarbonyl and the substituted lower alkylsulfonyl, the halogen has the same meaning as (i) defined above; the lower alkyl and the lower alkyl moiety of the lower alkoxy, the lower alkoxycarbonyl and the N-(lower alkanoyl)-N-(lower alkyl)amino

have the same meanings as (ii) defined above; the alkylene moiety of the aralkyl has the same meaning as (iii) defined above; the aryl and the aryl moiety of the aralkyl, the aroyl and the arylsulfonyl have the same meanings as (vi) defined above; the lower alkanoyl and the lower alkanoyl moiety of the lower alkanoylamino and the N-(lower alkanoyl)-N-(lower alkyl)amino have the same meanings as (vii) defined above; the heterocyclic group has the same meaning as (viii) defined above; the heterocyclic group formed together with the adjacent nitrogen atom has the same meaning as (ix) defined above; and the heteroaroyl has the same meaning as (x) defined above.

(xii) Examples of the substituents in the substituted aryl, the substituted aroyl, the substituted aralkyl, the substituted arylsulfonyl, the substituted heteroaroyl, the substituted heterocyclic group and the substituted heterocyclic group formed together with the adjacent nitrogen atom, which may be the same or different and is 1 to 3 in number, include

(xii-a) halogen,
(xii-b) nitro,
(xii-c) nitroso,
(xii-d) carboxy,
(xii-e) substituted or unsubstituted lower alkyl [the substituent(s) in the substituted lower alkyl has the same meaning as (xi) defined above],
(xii-f) substituted or unsubstituted lower alkenyl [the substituent(s) in the substituted lower alkenyl has the same meaning as (xi) defined above],
(xii-g) substituted or unsubstituted lower alkynyl [the substituent(s) in the substituted lower alkynyl has the same meaning as (xi) defined above],
(xii-h) substituted or unsubstituted lower alkoxycarbonyl [the substituent(s) in the substituted lower alkoxycarbonyl has the same meaning as (xi) defined above],
(xii-i) substituted or unsubstituted lower alkanoyl [the substituent(s) in the substituted lower alkanoyl has the same meaning as (xi) defined above],
(xii-j) substituted or unsubstituted aryl [the substituent(s) in the substituted aryl, which is 1 to 3 in number, is for example, halogen, hydroxy, nitro, cyano, carboxy, lower alkanoyl, lower alkoxycarbonyl, aralkyl, aroyl, substituted or unsubstituted lower alkyl (the substituent(s) in the substituted lower alkyl, which is 1 to 3 in number, is for example hydroxy or the like), substituted or unsubstituted lower alkoxy (the substituent(s) in the substituted lower alkoxy, which is 1 to 3 in number, is for example hydroxy or the like) or the like],
(xii-k) $NR^{17a}R^{17b}$ {wherein $R^{17a}$ and $R^{17b}$ may be the same or different and each represents a hydrogen atom, lower alkyl sulfonyl, substituted or unsubstituted lower alkyl [the substituent(s) in the substituted lower alkyl has the same meaning as (xi) defined above], substituted or unsubstituted lower alkenyl [the substituent(s) in the substituted lower alkenyl has the same meaning as (xi) defined above], substituted or unsubstituted lower alkynyl [the substituent(s) in the substituted lower alkynyl has the same meaning as (xi) defined above], substituted or unsubstituted lower alkoxy [the substituent(s) in the substituted lower alkoxy has the same meaning as (xi) defined above], substituted or unsubstituted lower alkanoyl [the substituent(s) in the substituted lower alkanoyl has the same meaning as (xi) defined above], substituted or unsubstituted aryl [the substituent(s) in the substituted aryl, which is 1 to 3 in number, is for example, halogen, hydroxy, nitro, cyano, carboxy, lower alkanoyl, lower alkoxycarbonyl, aralkyl, aroyl, substituted or unsubstituted lower alkyl (the substituent(s) in the substituted lower alkyl, which is 1 to 3 in number, is for example hydroxy or the like), substituted or unsubstituted lower alkoxy (the substituent(s) in the substituted lower alkoxy, which is 1 to 3 in number, is for example hydroxy or the like) or the like], substituted or unsubstituted aroyl [the substituent(s) in the substituted aroyl, which is 1 to 3 in number, is for example, halogen, hydroxy, nitro, cyano, carboxy, lower alkanoyl, lower alkoxycarbonyl, aralkyl, aroyl, substituted or unsubstituted lower alkyl (the substituent(s) in the substituted lower alkyl, which is 1 to 3 in number, is for example hydroxy or the like), substituted or unsubstituted lower alkoxy (the substituent (s) in the substituted lower alkoxy, which is 1 to 3 in number, is for example hydroxy or the like) or the like] or a substituted or unsubstituted heterocyclic group [the substituent(s) in the substituted heterocyclic group, which is 1 to 3 in number, is for example, halogen, hydroxy, nitro, cyano, carboxy, lower alkanoyl, lower alkoxycarbonyl, aralkyl, aroyl, substituted or unsubstituted lower alkyl (the substituent(s) in the substituted lower alkyl, which is 1 to 3 in number, is for example hydroxy or the like), substituted or unsubstituted lower alkoxy (the substituent (s) in the substituted lower alkoxy, which is 1 to 3 in number, is for example hydroxy or the like) or the like], or $R^{17a}$ and $R^{17b}$ are combined together with the adjacent nitrogen atom thereto to form a substituted or unsubstituted heterocyclic group [the substituent(s) in the substituted heterocyclic group formed with the adjacent nitrogen atom, which is 1 to 3 in number, is for example, halogen, amino, nitro, hydroxy, oxo, cyano, carboxy, lower alkoxycarbonyl, aralkyl, aroyl, heteroaroyl, substituted or unsubstituted lower alkyl (the substituent(s) in the substituted lower alkyl, which is 1 to 3 in number, is for example, hydroxy, lower alkoxy or the like), substituted or unsubstituted lower alkoxy (the substituent(s) in the substituted lower alkoxy, which is 1 to 3 in number, is for example, hydroxy, lower alkoxy or the like), substituted or unsubstituted lower alkanoyl (the substituent(s)

in the substituted lower alkanoyl, which is 1 to 3 in number, is for example, amino, hydroxy, lower alkoxy, lower alkanoylamino, N-(lower alkanoyl)-N-(lower alkyl)amino or the like), substituted or unsubstituted heteroalicyclic-carbonyl (the substituent(s) in the substituted heteroalicyclic-carbonyl, which is 1 to 3 in number, is for example, halogen, hydroxy, oxo, lower alkyl, lower alkoxy or the like) or the like]},

(xii-l) $CONR^{18a}R^{18b}$ (wherein $R^{18a}$ and $R^{18b}$ have the same meanings as $R^{17a}$ and $R^{17b}$ defined above, respectively),

(xii-m) $OR^{19}$ {wherein $R^{19}$ represents a hydrogen atom, substituted or unsubstituted lower alkyl [the substituent (s) in the substituted lower alkyl has the same meaning as (xi) defined above], substituted or unsubstituted aryl [the substituent(s) in the substituted aryl, which is 1 to 3 in number, is for example, halogen, hydroxy, nitro, cyano, carboxy, lower alkanoyl, lower alkoxycarbonyl, aralkyl, aroyl, substituted or unsubstituted lower alkyl (the substituent(s) in the substituted lower alkyl, which is 1 to 3 in number, is for example hydroxy or the like), substituted or unsubstituted lower alkoxy (the substituent(s) in the substituted lower alkoxy, which is 1 to 3 in number, is for example hydroxy or the like) or the like] or a substituted or unsubstituted heterocyclic group [the substituent(s) in the substituted heterocyclic group, which is 1 to 3 in number, is for example, halogen, hydroxy, nitro, cyano, carboxy, lower alkanoyl, lower alkoxycarbonyl, aralkyl, aroyl, substituted or unsubstituted lower alkyl (the substituent(s) in the substituted lower alkyl, which is 1 to 3 in number, is for example hydroxy or the like), substituted or unsubstituted lower alkoxy (the substituent(s) in the substituted lower alkoxy, which is 1 to 3 in number, is for example hydroxy or the like) or the like] or the like },

(xii-n) heteroaroyl,

(xii-o) substituted or unsubstituted heteroalicyclic-carbonyl [the substituent(s) in the substituted heteroalicyclic-carbonyl, which is 1 to 3 in number, is for example, halogen, hydroxy, oxo, lower alkyl, lower alkoxy or the like], or the like.

The substituent(s) in the substituted heterocyclic group, and the substituent(s) in the substituted heterocyclic group formed with the adjacent nitrogen atom may be, in addition to (xii-a) to (xii-o), the following (xii-p) or (xii-q):

(xii-p) oxo

(xii-q) $-O(CR^{20a}R^{20b})_{na}O-$ (wherein $R^{20a}$ and $R^{20b}$ may be the same or different and each represents a hydrogen atom or lower alkyl, na represents 2 or 3, and the two terminal oxygen atoms are combined to the same carbon atom in the heterocyclic group of the substituted heterocyclic group or the substituted heterocyclic group formed with the adjacent nitrogen atom).

In the definition of the substituents (xii) in the substituted aryl, the substituted aroyl, the substituted aralkyl, the substituted arylsulfonyl, the substituted heteroaroyl, the substituted heterocyclic group and the substituted heterocyclic group formed with the adjacent nitrogen atom, the halogen has the same meaning as (i) defined above; the lower alkyl and the lower alkyl moiety of the lower alkoxy, the lower alkoxycarbonyl and the lower alkylsulfonyl have the same meanings as (ii) defined above; the alkylene moiety of the aralkyl has the same meaning as (iii) defined above; the lower alkenyl has the same meaning as (iv) defined above; the lower alkynyl has the same meaning as (v) defined above; the aryl and the aryl moiety of the aroyl and the aralkyl have the same meanings as (vi) defined above; the lower alkanoyl and the lower alkanoyl moity of the lower alkanoylamino and the N-(lower alkanoyl)-N-(lower alkyl)amino have the same meaning as (vii) defined above; the heterocyclic group has the same meaning as (viii) defined above; the heteroalicyclic moiety of the heteroalicyclic-carbonyl has the same meaning as (viii-b) defined above; the heterocyclic group formed with the adjacent nitrogen atom has the same meaning as (ix) defined above; the heteroaroyl has the same meaning as (x) defined above.

Examples of the pharmaceutically acceptable salts of Compound (I), (Ia), (Ib), (Ib-1), (Ib-2) and (Ic) include, for example, pharmaceutically acceptable acid addition salts, metal salts, ammonium salts, organic amine addition salts, amino acid addition salts and the like. The acid addition salts include, for example, inorganic acid salts such as hydrochlorides, sulfates and phosphates; and organic acid salts such as acetate, maleate, fumarate, tartrates, citrates, lactates, aspartates, and glutamates. The metal salts include, for example, alkali metal salts such as sodium salts and potassium salts; alkaline earth metal salts such as magnesium salts and calcium salts; as well as aluminum salts, zinc salts and the like. The ammonium salts include, for example, salts of ammonium, tetramethylammonium and the like. The organic amine addition salts include, for example, morpholine salts, piperidine salts and the like. The amino acid addition salts include, for example, lysine salts, glycine salts, phenylalanine salts and the like.

When it is desired to obtain salts of Compound (I), (Ia), (Ib), (Ib-1), (Ib-2) and (Ic), the salt may be purified as it is, if Compound (I), (Ia), (Ib), (Ib-1), (Ib-2) and (Ic) are obtained in a form of a salt; and if Compound (I), (Ia), (Ib), (Ib-1), (Ib-2) and (Ic) is obtained in a free form, it is dissolved or suspended in an appropriate solvent followed by adding an acid or a base thereto to form a salt.

There can be isomers such as positional isomers, geometrical isomers or optical isomers in Compound (I), (Ia), (Ib), (Ib-1), (Ib-2) and (Ic). All possible isomers including these isomers, and mixtures of the isomers in any ratio can be used as Abl kinase inhibitor of the present invention.

Compound (I), (Ia), (Ib), (Ib-1), (Ib-2) and (Ic) or the pharmaceutically acceptable salts thereof may exist in a form of adducts to water or various solvents. These adducts can also be used as Abl kinase inhibitors of the present inhibition.

Abl kinase inhibitors of the present invention include inhibitors to various mutants such as Abl kinase having Abl T315I mutation.

The disease associated with Abl kinase includes, for example, cancer of hematopoietic tumor, mammary cancer, uterine body cancer, uterine cervix cancer, prostatic cancer, bladder cancer, renal cancer, gastric cancer, esophageal cancer, hepatic cancer, biliary tract cancer, colon cancer, rectal cancer, pancreatic cancer, lung cancer, oral cavity and pharynx cancer, osteosarcoma, melanoma, brain neoplasm, and the like.

The hematopoietic tumor refers to tumors typically in hemocytes. Examples of pathosis based on the hematopoietic tumor include leukemia such as chronic myeloid leukemia, acute myeloid leukemia, chronic lymphoid leukemia and acute lymphoid leukemia; myeloma such as multiple myeloma; lymphoma; and the like.

Compounds (I), (Ia), (Ib), (Ib-1), (Ib-2) and (Ic) of the present invention can be synthesized, for example, by the method discribed in WO2005/012257, W02005/012258 and the like.

Example of the compounds (I), (Ia), (Ib), (Ib-1), (Ib-2) and (Ic) used in the present invention include compounds described in the Table 1-1 and 1-2. In the Table 1-1 and 1-2, Me represents methyl.

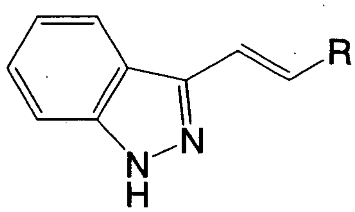

Table 1-1

| Compound No. | R | salt |
|---|---|---|
| 1 | 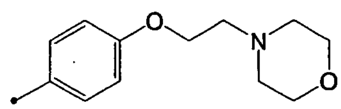 | HCl |
| 2 | 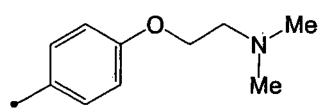 | HCl |
| 3 | 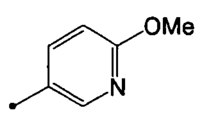 | |
| 4 | 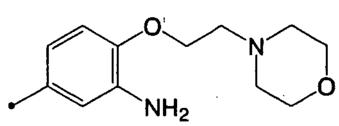 | |
| 5 | 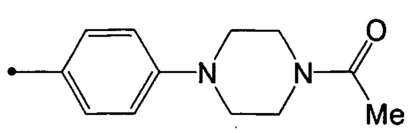 | |

(continued)

| Compound No. | R | salt |
|---|---|---|
| 6 | 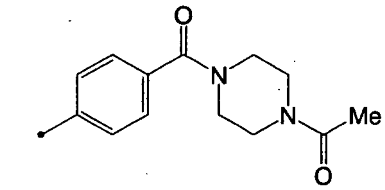 | |
| 7 | 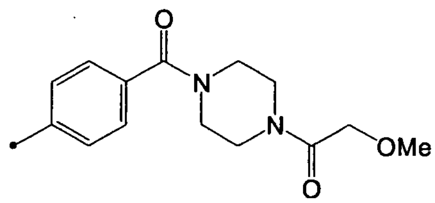 | |

Table 1-2

| | | |
|---|---|---|
| 8 | 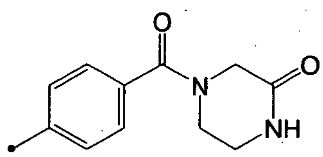 | |
| 9 | 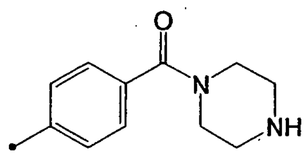 | |
| 10 | 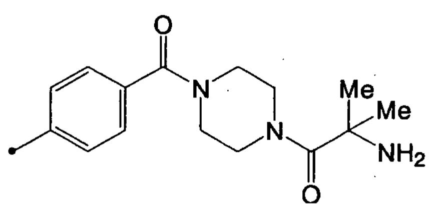 | HCl |
| 11 | 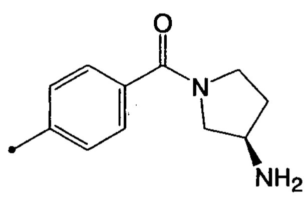 | |
| 12 | 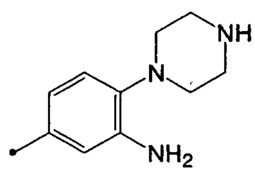 | |
| 13 | 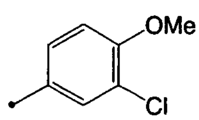 | |

(continued)

| | | |
|---|---|---|
| 14 | 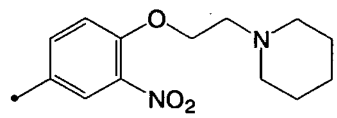 | |

Test example 1: Inhibitory activity on Abl kinase having Abl T315I mutation (Abl T315I inhibitory activity)

In order to measure Abl T315I inhibitory activity, the following method was used: A reaction solution was prepared so that the final concentrations in a volume of 25 μL were 5 to 10 mU of Abl(T315I) protein (Upstate, catalog No. 14-522), 8 mmol/L of 3-morpholinopropane sulfonic acid (pH 7.0), 0.2 mmol/L of EDTA, 50 μmol/L of EAIYAAP-FAKKK, 10 mmol/L of Mg acetate, [$\gamma$-$^{33}$P-ATP] (500 cpm/pmol), 0.1% of dimethyl sulfoxide (DMSO), and 1 μmol/L of test compound, followed by enzyme reaction at room temperature for 40 minutes. The reaction was terminated with 5 μL of a 3% aqueous phosphoric acid solution, and the reaction solution was added to a P30 filter mat. Then, the filter was washed with a 75 mmol/L aqueous phosphoric acid solution three times and 100% methanol once, and then dried. Then, the radioactivity of the filter was measured.

Assuming that a measured value of an enzyme to which 0.1% of DMSO was added was 100%, and a measured value of a sample to which an enzyme was not added was 0%, the relative activity (%) of radioactivity of a sample to which the test compound was added was calculated, and a value obtained by subtracting the calculated value from 100 was considered as Abl T315I inhibitory activity (%) of the test compound.

Compounds 4, 6, 7, 8, 9, 10, and 11 exhibited an Abl T315I inhibitory activity of 90% or more at a concentration of 1 μmol/L. This result indicates that the compound (I) shows effective Abl T315I inhibitory activity.

Test example 2: Cell growth-suppressing activity on human chronic myeloid leukemia cell strain having imatinib mesylate resistance

The cell growth suppression rate of a test compound for human chronic myeloid leukemia cell strain (TCC-Y/sr) was measured by the following method:

TCC-Y/sr cells are a cell strain of human chronic myeloid leukemia which was rendered resistant to imatinib mesylate due to the gene mutation in which a threonine residue at the 315-position in the amino acid sequence of Abl kinase possessed by the cells was substituted by isoleucine (Patent No. 3550565). The TCC-Y/sr cells were cultured using Roswell Park Memorial Institute's Medium (RPMI) 1640 medium (GIBCO) containing 10% of fetal calf serum (FCS).

The TCC-Y/sr cells were inoculated at 5000 cells/well in TC MICROWELL 96U plate (Nalge Nunc In.) in an amount of 50 μL for each well and cultured at 37°C for 24 hours in a 5% carbon dioxide gas incubator. To each well, 50 μL of a solution containing a test compound prepared by stepwise dilution with the incubation medium of the respective cells was added, and the resulting mixture was again incubated at 37°C for 72 hours in a 5% carbon dioxide gas incubator. Then, 50 μL of XTT reagent {sodium 3-[1-(phenylaminocarbonyl)-3,4-tetrzolium]-bis(4-methoxy-6-nitro)benzene sulfonic acid hydrate} (Roche Diagnostics Ltd.) was added to the medium and incubated at 37°C for 4 hours. Then, absorbance was measured at 492 nm (control wavelength 690 nm) using microplate spectrophotometer SPECTRA max 340PC (Molecular Devices Corporation). Assuming that a value of a well in which the solvent of the test compound solution was added and cultured was 100%, the cell growth suppression rate was calculated by the equation below. In addition, an absorbance value measured for a sample to which XTT was added immediately after the addition of the solvent was used as a blank.

$$\text{Cell growth suppression rate} = \frac{\text{absorbance of well to which test compound was added} - \text{absorbance of blank}}{\text{absorbance of well to which solvent was added} - \text{absorbance of blank}} \times 100$$

Among compounds (I) of the present invention, the compounds undergoing the test exhibited a growth-suppressing activity of 50% or more at a concentration of 1 μmol/L. This result indicates that the compound (I) of the present invention exhibits cell growth-suppressing activity on the human chronic myeloid leukemia cell strain TCC-Y/sr having resistance to imatinib mesylate.

Compounds (I), (Ia), (Ib), (Ib-1), (Ib-2) and (Ic), or pharmaceutically acceptable salts thereof may be used as it is or in various pharmaceutical forms depending upon the pharmacological effect, purpose of administration, etc. A

pharmaceutical composition of the present invention can be manufactured by uniform mixing of Compounds (I), (Ia), (Ib), (Ib-1), (Ib-2) and (Ic), or pharmaceutically acceptable salts thereof in an amount which is effective as an active ingredient with pharmaceutically acceptable carriers. These carriers can have forms in a wide range according to desired dosage form for administration. It is preferred that the pharmaceutical composition is in a unit dosage form for oral administration or parental administration such as injection.

In the manufacture of tablets, excipient such as lactose and mannitol, disintegrator such as starch, lubricant such as magnesium stearate, binder such as polyvinyl alcohol and hydroxypropyl cellulose, and surfactant such as sucrose fatty acid esters and sorbitol fatty acid esters, etc. may be used in accordance with a conventional procedure. Tablets containing 1 to 200 mg of an active ingredient per tablet are preferred.

In the manufacture of injections, water, physiological saline, vegetable oil such as olive oil and peanut oil, solvent such as ethyl oleate and propylene glycol, dissolving agent such as sodium benzoate, sodium salicylate and urethane, isotonizing agent such as sodium chloride and glucose, preservative such as phenol, cresol, p-hydroxybenzoate and chlorobutanol, and antioxidant such as ascorbic acid and sodium pyrosulfite, etc. may be used by a conventional procedure.

Compounds (I), (Ia), (Ib), (Ib-1), (Ib-2) and (Ic), or pharmaceutically acceptable salts thereof can be administered either orally or parentally by means of injection solution, etc. The effective dose and frequency of administration vary depending on the dosage form, age, body weight and symptom of a patient, etc. In general, Compounds (I), (Ia), (Ib), (Ib-1), (Ib-2) and (Ic), or pharmaceutically acceptable salts thereof may preferably be administered in an amount of 0.01 to 100 mg/kg per day.

Compounds 1, 2, 3, 4, 5, 7, 8, 9, 10, 12, 13 and 14 used in the present invention can be synthesized according to examples 5, 2, 22, 38, 54, 69, 70, 64, 74, 59, 51 and 29 of W02005/012258, respectively. Compounds 6 and 11 can be synthesized according to examples 13 and 158 of W02005/012257, respectively.

Example 1

Formulation Example 1: Tablet

Tablets having the following composition are prepared by a usual method.

250 g of Compound 4, 1598.5 g of mannitol, 100 g of sodium carboxylmethyl starch, 10 g of light anhydrous silicic acid, 40 g of magnesium stearate, and 1.5 g of yellow iron sesquioxide are mixed by an usual method. The resultant mixture is tableted with a tableting machine (Manufactured by Kikusui Seisakusho Ltd., Purepress Correct-12 model) having a punch and die of 8 mm in diameter to prepare tablets (containing 25 mg of an active component per tablet).

| Prescription | |
|---|---|
| Compound 4 | 25 mg |
| Mannitol | 159.85 mg |
| Sodium carboxylmethyl starch | 10 mg |
| Light anhydrous silicic acid | 1 mg |
| Magnesium stearate | 4 mg |
| Yellow iron sesquioxide | 0.15 mg |
| | 200 mg |

Example 2

Formulation Example 2: Injection

Injections having the following composition are prepared by a usual method.

Compound 6 (1 g) and 5 g of D-mannitol are added to distilled water for injection and mixed, and further hydrochloric acid and an aqueous sodium hydroxide solution are added to the mixture to adjust pH to 6. Then, distilled water is added to a total of 1000 mL. Each glass vial is aseptically filled with 2 mL of the resultant mixture to prepare injections (containing 2 mg of an active component per vial).

| Prescription | |
|---|---|
| Compound 6 | 2 mg |
| D-mannitol | 10 mg |

(continued)

| Prescription | |
|---|---|
| Hydrochloric acid | proper amount |
| Aqueous sodium hydroxide solution | proper amount |
| Distilled water for injection | proper amount |
| | 2.00 mL |

Industrial Applicability

The present invention provides an Abl kinase inhibitor which comprises, as an active ingredient, an indazole derivative or a pharmaceutically acceptable salt thereof, and the like.

**Claims**

1. An Abelson kinase (Abl kinase) inhibitor which comprises, as an active ingredient, an indazole derivative represented by Formula (I)

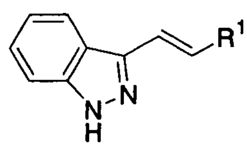

(I)

(wherein $R^1$ represents substituted or unsubstituted aryl or a substituted or unsubstituted heterocyclic group) or a pharmaceutically acceptable salt thereof.

2. An Abelson kinase (Abl kinase) inhibitor which comprises, as an active ingredient, an indazole derivative represented by Formula (Ia)

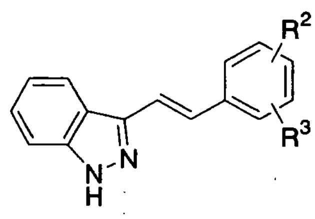

(Ia)

[wherein $R^2$ represents $CONR^{4a}R^{4b}$ (wherein $R^{4a}$ and $R^{4b}$ may be the same or different and each represents a hydrogen atom, substituted or unsubstituted lower alkyl, substituted or unsubstituted aryl, substituted or unsubstituted aralkyl, or a substituted or unsubstituted heterocyclic group, or $R^{4a}$ and $R^{4b}$ are combined together with the adjacent nitrogen atom thereto to form a substituted or unsubstituted heterocyclic group) or $NR^{5a}R^{5b}$ (wherein $R^{5a}$ represents substituted or unsubstituted lower alkylsulfonyl or substituted or unsubstituted arylsulfonyl and $R^{5b}$ represents a hydrogen atom or substituted or unsubstituted lower alkyl) and $R^3$ represents a hydrogen atom, halogen, cyano, nitro, hydroxy, carboxy, lower alkoxycarbonyl, substituted or unsubstituted lower alkyl, substituted or unsubstituted lower alkoxy, substituted or unsubstituted lower alkanoyl, $CONR^{6a}R^{6b}$ (wherein $R^{6a}$ and $R^{6b}$ may be the same or different and each represents a hydrogen atom, substituted or unsubstituted lower alkyl, substituted or unsubstituted aryl, substituted or unsubstituted aralkyl or a substituted or unsubstituted heterocyclic group, or $R^{6a}$ and $R^{6b}$ are combined together with the adjacent nitrogen atom thereto to form a substituted or unsubstituted heterocyclic group) or $NR^{7a}R^{7b}$ (wherein $R^{7a}$ and $R^{7b}$ may be the same or different and each represents a hydrogen atom, substituted or unsubstituted lower alkyl, substituted or unsubstituted lower alkanoyl, substituted or unsubstituted aroyl, substituted or unsubstituted heteroaroyl, substituted or unsubstituted aralkyl, substituted or unsubstituted lower alkylsulfonyl or substituted or unsubstituted arylsulfonyl)], or a pharmaceutically acceptable salt thereof.

3. The Abelson kinase (Abl kinase) inhibitor according to Claim 2, wherein $R^2$ is $CONR^{4a}R^{4b}$ (wherein $R^{4a}$ and $R^{4b}$ have the same meanings as defined above, respectively) and $R^3$ is a hydrogen atom.

4. The Abelson kinase (Abl kinase) inhibitor according to Claim 2, wherein $R^2$ is $NR^{5a}R^{5b}$ (wherein $R^{5a}$ and $R^{5b}$ have the same meanings as defined above, respectively) and $R^3$ is substituted or unsubstituted lower alkoxy.

5. The Abelson kinase (Abl kinase) inhibitor according to Claim 2, wherein $R^2$ is $NR^{5a}R^{5b}$ (wherein $R^{5a}$ and $R^{5b}$ have the same meanings as defined above, respectively) and $R^3$ is a hydrogen atom.

6. An Abelson kinase (Abl kinase) inhibitor which comprises, as an active ingredient, an indazole derivative represented by Formula (Ib)

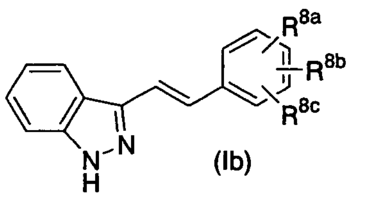

(Ib)

[wherein $R^{8a}$, $R^{8b}$ and $R^{8c}$ may be the same or different and each represents a hydrogen atom, halogen, nitro, nitroso, carboxy, cyano, substituted or unsubstituted lower alkyl, substituted or unsubstituted lower alkanoyl, substituted or unsubstituted lower alkoxycarbonyl, substituted or unsubstituted aryl, $NR^{9a}R^{9b}$ (wherein $R^{9a}$ and $R^{9b}$ may be the same or different and each represents a hydrogen atom, substituted or unsubstituted lower alkyl, substituted or unsubstituted lower alkenyl, substituted or unsubstituted lower alkynyl, substituted or unsubstituted lower alkoxy, substituted or unsubstituted lower alkanoyl, substituted or unsubstituted aryl, substituted or unsubstituted aroyl, a substituted or unsubstituted heterocyclic group or substituted or unsubstituted heteroaroyl, or $R^{9a}$ and $R^{9b}$ are combined together with the adjacent nitrogen atom thereto to form a substituted or unsubstituted heterocyclic group) or $OR^{10}$ (wherein $R^{10}$ represents a hydrogen atom, substituted or unsubstituted lower alkyl, substituted or unsubstituted aryl, substituted or unsubstituted aroyl or a substituted or unsubstituted heterocyclic group)], or a pharmaceutically acceptable salt thereof.

7. The Abelson kinase (Abl kinase) inhibitor according to Claim 6, wherein at least one of $R^{8a}$, $R^{8b}$ and $R^{8c}$ is $NR^{9a}R^{9b}$ (wherein $R^{9a}$ and $R^{9b}$ have the same meanings as defined above, respectively).

8. The Abelson kinase (Abl kinase) inhibitor according to Claim 6, wherein at least one of $R^{8a}$, $R^{8b}$ and $R^{8c}$ is $NR^{9c}R^{9d}$ (wherein $R^{9c}$ and $R^{9d}$ may be the same or different and each represents a hydrogen atom or substituted or unsubstituted lower alkanoyl, or $R^{9c}$ and $R^{9d}$ are combined together with the adjacent nitrogen atom thereto to form a substituted or unsubstituted heterocyclic group).

9. The Abelson kinase (Abl kinase) inhibitor according to Claim 6, wherein at least one of $R^{8a}$, $R^{8b}$ and $R^{8c}$ is $OR^{10}$ (wherein $R^{10}$ has the same meaning as defined above).

10. The Abelson kinase (Abl kinase) inhibitor according to Claim 6, wherein at least one of $R^{8a}$, $R^{8b}$ and $R^{8c}$ is $OR^{10a}$ (wherein $R^{10a}$ represents substituted or unsubstituted lower alkyl).

11. An Abelson kinase (Abl kinase) inhibitor which comprises, as an active ingredient, an indazole derivative represented by Formula (Ib-1)

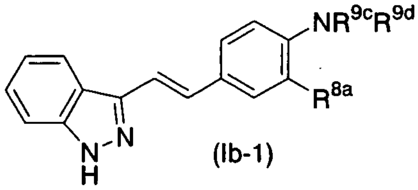

(Ib-1)

(wherein $R^{8a}$, $R^{9c}$ and $R^{9d}$ have the same meanings as defind above, respectively) or a pharmaceutically acceptable salt thereof.

**12.** An Abelson kinase (Abl kinase) inhibitor which comprises, as an active ingredient, an indazole derivative represented by Formula (Ib-2)

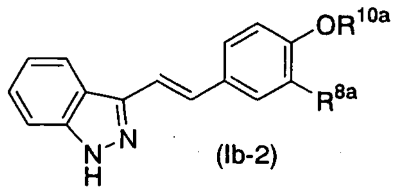

(Ib-2)

(wherein $R^{8a}$ and $R^{10a}$ have the same meanings as defind above, respectively) or a pharmaceutically acceptable salt thereof.

**13.** An Abelson kinase (Abl kinase) inhibitor which comprises, as an active ingredient, an indazole derivative represented by Formula (Ic)

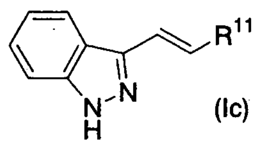

(Ic)

{wherein $R^{11}$ represents a substituted or unsubstituted heterocyclic group [substituent(s) in the substituted heterocyclic group may be the same or different, are 1 to 3 in number, and are oxo, formyl, carboxy, lower alkoxycarbonyl, substituted or unsubstituted lower alkyl, substituted or unsubstituted lower alkoxy, $CONR^{12a}R^{12b}$ (wherein $R^{12a}$ and $R^{12b}$ may be the same or different and each represents a hydrogen atom or substituted or unsubstituted lower alkyl), $NR^{13a}R^{13b}$ (wherein $R^{13a}$ and $R^{13b}$ may be the same or different and each represents a hydrogen atom, lower alkanoyl, lower alkoxycarbonyl, aralkyl, substituted or unsubstituted lower alkyl, substituted or unsubstituted aryl, substituted or unsubstituted aroyl or a substituted or unsubstituted heterocyclic group) or - O $(CR^{14a}R^{14b})_nO$- (wherein $R^{14a}$ and $R^{14b}$ may be the same or different and each represents a hydrogen atom or lower alkyl, n represents 2 or 3, and two terminal oxygen atoms are bonded to the same carbon atom in the heterocyclic group of the substituted heterocyclic group)]} or a pharmaceutically acceptable salt thereof.

**14.** The Abelson kinase (Abl kinase) inhibitor according to Claim 13, wherein substituents in the substituted heterocyclic group are amino, oxo, lower alkoxy, lower alkoxycarbonylamino, aroylamino or lower alkoxycarbonyl-substituted lower alkyl.

**15.** The Abelson kinase (Abl kinase) inhibitor according to Claim 13, wherein $R^{11}$ is 3-pyridyl.

**16.** A therapeutic agent for a disease associated with Abelson kinase (Abl kinase) which comprises, as an active ingredient, the indazole derivative or the pharmaceutically acceptable salt thereof described in any one of Claims 1 to 15.

**17.** The therapeutic agent according to Claim 16, wherein Abelson kinase (Abl kinase) is Abl kinase which has point mutation in the amino acid sequence thereof.

**18.** The therapeutic agent according to Claim 16, wherein Abelson kinase (Abl kinase) is Abl kinase which has gene mutation in which a threonine residue at the 315-position is substituted with isoleucine in the amino acid sequence thereof (Abl T315I).

**19.** The therapeutic agent according to any one of Claims 16 to 18, wherein a disease associated with Abelson kinase

(Abl kinase) is cancer.

20. The therapeutic agent according to Claim 19, wherein cancer is cancer derived from hematopoietic tumor, mammary cancer, uterine body cancer, uterine cervix cancer, prostatic cancer, bladder cancer, renal cancer, gastric cancer, esophageal cancer, hepatic cancer, biliary tract cancer, colon cancer, rectal cancer, pancreatic cancer, lung cancer, oral cavity and pharynx cancer, osteosarcoma, melanoma or brain neoplasm.

21. The therapeutic agent for cancer according to Claim 19, wherein cancer is leukemia, myeloma or lymphoma.

22. The therapeutic agent for cancer according to Claim 19, wherein cancer is chronic myeloid leukemia.

23. The therapeutic agent for cancer according to Claim 19, wherein cancer is leukemia having acquired resistance to imatinib.

24. The therapeutic agent for cancer according to Claim 19, wherein cancer is chronic myeloid leukemia having acquired resistance to imatinib.

25. A method for inhibiting Abelson kinase (Abl kinase) comprising a step of administering an effective amount of the indazole derivative or the pharmaceutically acceptable salt thereof described in any one of Claims 1 to 15.

26. A method for treating a disease associated with Abelson kinase (Abl kinase) comprising a step of administering an effective amount of the indazole derivative or the pharmaceutically acceptable salt thereof described in any one of Claims 1 to 15.

27. The treating method according to Claim 26, wherein Abelson kinase (Abl kinase) is Abl kinase which has point mutation in the amino acid sequence thereof.

28. The treating method according to Claim 26, wherein Abelson kinase (Abl kinase) is Abl kinase which has gene mutation in which a threonine residue at the 315-position is substituted with isoleucine in the amino acid sequence thereof (Abl T315I).

29. The method for treating cancer comprising a step of administering an effective amount of the indazole derivative or the pharmaceutically acceptable salt thereof described in any one of Claims 1 to 15.

30. The treating method according to Claim 29, wherein cancer is cancer derived from hematopoietic tumor, mammary cancer, uterine body cancer, uterine cervix cancer, prostatic cancer, bladder cancer, renal cancer, gastric cancer, esophageal cancer, hepatic cancer, biliary tract cancer, colon cancer, rectal cancer, pancreatic cancer, lung cancer, oral cavity and pharynx cancer, osteosarcoma, melanoma or brain neoplasm.

31. The treating method according to Claim 29, wherein cancer is leukemia, myeloma or lymphoma.

32. The treating method according to Claim 29, wherein cancer is chronic myeloid leukemia.

33. The treating method according to Claim 29, wherein cancer is leukemia having acquired resistance to imatinib.

34. The treating method according to Claim 29, wherein cancer is chronic myeloid leukemia having acquired resistance to imatinib.

35. Use of the indazole derivative or the pharmaceutically acceptable salt thereof described in any one of Claims 1 to 15 for the manufacture of Abelson kinase (Abl kinase) inhibitor.

36. Use of the indazole derivative or the pharmaceutically acceptable salt thereof described in any one of Claims 1 to 15 for the manufacture of therapeutic agent for a disease associated with Abelson kinase (Abl kinase) inhibitor.

37. The use according to Claim 36, wherein Abelson kinase (Abl kinase) is Abl kinase which has point mutation in the amino acid sequence thereof.

38. The use according to Claim 36, wherein Abelson kinase (Abl kinase) is Abl kinase which has gene mutation in which

a threonine residue at the 315-position is substituted with isoleucine in the amino acid sequence thereof (Abl T315I).

**39.** Use of the indazole derivative or the pharmaceutically acceptable salt thereof described in any one of Claims 1 to 15 for the manufacture of a therapeutic agent for cancer.

**40.** The use according to Claim 39, wherein cancer is cancer derived from hematopoietic tumor, mammary cancer, uterine body cancer, uterine cervix cancer, prostatic cancer, bladder cancer, renal cancer, gastric cancer, esophageal cancer, hepatic cancer, biliary tract cancer, colon cancer, rectal cancer, pancreatic cancer, lung cancer, oral cavity and pharynx cancer, osteosarcoma, melanoma or brain neoplasm.

**41.** The use according to Claim 39, wherein cancer is leukemia, myeloma or lymphoma.

**42.** The use according to Claim 39, wherein cancer is chronic myeloid leukemia.

**43.** The use according to Claim 39, wherein cancer is leukemia having acquired resistance to imatinib.

**44.** The use according to Claim 39, wherein cancer is chronic myeloid leukemia having acquired resistance to imatinib.

**INTERNATIONAL SEARCH REPORT**

International application No.
PCT/JP2007/063095

A. CLASSIFICATION OF SUBJECT MATTER
*C07D231/56*(2006.01)i, *A61K31/416*(2006.01)i, *A61K31/4439*(2006.01)i, *A61K31/454*(2006.01)i, *A61K31/496*(2006.01)i, *A61K31/5377*(2006.01)i, *A61P35/00*(2006.01)i, *A61P35/02*(2006.01)i, *A61P43/00*(2006.01)i,
According to International Patent Classification (IPC) or to both national classification and IPC

B. FIELDS SEARCHED
Minimum documentation searched (classification system followed by classification symbols)
C07D231/56, A61K31/416, A61K31/4439, A61K31/454, A61K31/496, A61K31/5377, A61P35/00, A61P35/02, A61P43/00, C07D401/06, C07D403/10

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2007 |
| Kokai Jitsuyo Shinan Koho | 1971-2007 | Toroku Jitsuyo Shinan Koho | 1994-2007 |

Electronic data base consulted during the international search (name of data base and, where practicable, search terms used)
BIOSIS(STN), CAplus(STN), EMBASE(STN), MEDLINE(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| X | JP 2-032059 A (KYOWA HAKKO KOGYO KABUSHIKI KAISHA), 01 February, 1990 (01.02.90), Claims; test examples (1), (2); compound 63 (Family: none) | 1-24,35-44 |
| X | WO 2005/012257 A1 (KYOWA HAKKO KOGYO KABUSHIKI KAISHA), 10 February, 2005 (10.02.05), Claims; test examples; compounds 13, 18, 65, 89, 122, 131, 158 & EP 1652842 A1 | 1-24,35-44 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 06 August, 2007 (06.08.07) | 28 August, 2007 (28.08.07) |
| Name and mailing address of the ISA/ Japanese Patent Office | Authorized officer |
| Facsimile No. | Telephone No. |

Form PCT/ISA/210 (second sheet) (April 2005)

**INTERNATIONAL SEARCH REPORT**

International application No. PCT/JP2007/063095

C (Continuation). DOCUMENTS CONSIDERED TO BE RELEVANT

| Category* | Citation of document, with indication, where appropriate, of the relevant passages | Relevant to claim No. |
|---|---|---|
| X | WO 2005/012258 A1 (KYOWA HAKKO KOGYO KABUSHIKI KAISHA), 10 February, 2005 (10.02.05), Claims; test examples; compounds 2, 5, 22, 29, 38, 51, 54, 59, 64, 69, 70, 74 & EP 1650194 A1 & US 2006/281789 A1 | 1-24,35-44 |
| P,X | WO 2006/080450 A1 (KYOWA HAKKO KOGYO KABUSHIKI KAISHA), 03 August, 2006 (03.08.06), Claims; test examples (Family: none) | 1-24,35-44 |
| P,X | WO 2006/118257 A1 (KYOWA HAKKO KOGYO KABUSHIKI KAISHA), 09 November, 2006 (09.11.06), Claim 8; Par. No. [0001]; referential examples (Family: none) | 1-24,35-44 |
| P,X | JP 2007-051082 A (KYOWA HAKKO KOGYO KABUSHIKI KAISHA), 01 March, 2007 (01.03.07), Claim 10; Par. No. [0001]; examples 4, 5 (Family: none) | 1-24,35-44 |
| P,X | WO 2007/058626 A1 (S BIO PTE LTD.), 24 May, 2007 (24.05.07), Claims 1 to 2, 4 to 11, 13 to 15, 67 to 77 (Family: none) | 1-24,35-44 |
| P,X | WO 2007/056075 A2 (TARGEGEN INC.), 18 May, 2007 (18.05.07), Claims 1, 3, 15, 48 to 52, 55 to 61, 63 to 69; examples 356, 358 & US 2007/149508 A1 & US 2007/161645 A1 & WO 2007/056023 A2 | 1-24,35-44 |
| A | JP 2006-501217 A (SUGEN INC.), 12 January, 2006 (12.01.06), Claims; Par. Nos. [0057], [0437] & WO 2004/014368 A1 & US 2004/092546 A1 & EP 1545515 A1 & US 7186716 B2 | 1-24,35-44 |
| A | JP 2005-507922 A (PHARMACIA CORP.), 24 March, 2005 (24.03.05), Claims; Par. No. [0070] & WO 2003/035625 A1 & US 2003/109550 A1 & EP 1427707 A1 | 1-24,35-44 |
| A | JP 2005-534635 A (PHARMACIA ITAL S.p.A.), 17 November, 2005 (17.11.05), Claims; Par. No. [0012] & WO 2003/097610 A1 & EP 1506176 A1 | 1-24,35-44 |

Form PCT/ISA/210 (continuation of second sheet) (April 2005)

**INTERNATIONAL SEARCH REPORT**

International application No.
PCT/JP2007/063095

C (Continuation). DOCUMENTS CONSIDERED TO BE RELEVANT

| Category* | Citation of document, with indication, where appropriate, of the relevant passages | Relevant to claim No. |
|---|---|---|
| A | US 2005/009876 A1 (BHAGWAT S S), 13 January, 2005 (13.01.05), Claims 1, 8 to 10, 12, 14 to 17; table 1; example 508 & US 2002/103229 A1 & EP 1313711 A2 & US 2004/077877 A1 & JP 2004-513882 A & US 2004/127536 A1 & US 2005/107457 A1 & US 6897231 B2 & US 2007/060616 A1 & US 7208513 B2 & US 7211594 B2 & WO 2002/010137 A2 | 1-24,35-44 |
| A | MADHUSUDAN, S and GANESAN, T.S., Tyrosine kinase inhibitors in cancer therapy, Clinical Biochemistry, July 2004, 37(7), pp. 618-635 Abstract | 1-24,35-44 |
| P,A | JP 2006-528225 A (HAEGERKVIST R P), 14 December, 2006 (14.12.06), Claims & WO 2004/105763 A2 & EP 1631291 A2 & US 2007/072932 A1 | 1-24,35-44 |

Form PCT/ISA/210 (continuation of second sheet) (April 2005)

**INTERNATIONAL SEARCH REPORT**

International application No.
PCT/JP2007/063095

Continuation of A. CLASSIFICATION OF SUBJECT MATTER
(International Patent Classification (IPC))

*C07D401/06*(2006.01)i, *C07D403/10*(2006.01)i

(According to International Patent Classification (IPC) or to both national classification and IPC)

Form PCT/ISA/210 (extra sheet) (April 2005)

**INTERNATIONAL SEARCH REPORT**

International application No.
PCT/JP2007/063095

**Box No. II Observations where certain claims were found unsearchable (Continuation of item 2 of first sheet)**

This international search report has not been established in respect of certain claims under Article 17(2)(a) for the following reasons:

1. ☒ Claims Nos.: 25-34
because they relate to subject matter not required to be searched by this Authority, namely:
Claims 25 to 34 pertain to methods for treatment of the human body by therapy and thus relate to a subject matter which this International Searching Authority is not required, under the provisions of Article 17(2)(a)(i) of the PCT and Rule 39.1(iv) of the Regulations under the PCT, to search.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.
2. ☐ As all searchable claims could be searched without effort justifying an additional fee, this Authority did not invite payment of any additional fee.
3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** the

☐ The additional search fees were accompanied by the applicant's protest and, where applicable, payment of a protest fee..

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2032059 A
- WO 0153268 A
- WO 0210137 A
- WO 0102369 A
- WO 02083648 A
- WO 03101968 A
- WO 2004094388 A
- WO 2004050088 A
- WO 20050137171 A
- WO 2005012257 A
- WO 2005012258 A
- WO 2005094823 A
- WO 3550565 A

### Non-patent literature cited in the description

- *Nature,* 1987, vol. 315, 550
- *NATURE,* 1987, vol. 325, 631
- *New England Journal of Medicine,* 2002, vol. 345, 645
- *Nature Review Drug Discovery,* 2004, vol. 3, 1001
- *Science,* 2001, vol. 293, 876
- *Cancer Cell,* 2005, vol. 7, 129
- *Science,* 2004, vol. 305, 399
- *Cancer Research,* 2005, vol. 65, 4500
- *Proceeding of the national academy of science of the united states of America,* 2005, vol. 102, 101011
- *Khimiya Geterotsiklicheskikh Soedinenii,* 1978, vol. 7, 957-959